# EUROPEAN PATENT SPECIFICATION

(11) **EP 1 944 028 B1**
(45) Date of publication and mention of the grant of the patent: **14.12.2011**
(21) Application number: 06830886.5
(22) Date of filing: 20.10.2006
(51) Int. Cl.: A61K 31/4515, A61K 31/445

(54) **SOLID ORAL FORMS OF EBASTINE**
FESTE ORALE FORMEN VON EBASTIN
FORMES ORALES SOLIDES D'EBASTINE

(30) Priority: 04.11.2005 ES 200502686
(43) Date of publication of application: 16.07.2008
(73) Proprietor: Simbec Iberica, SL, 08013 Barcelona (ES)
(72) Inventor: ROMA MILLAN, Jordi, E-08013 Barcelona (ES); MESTRE CASTELL, José, E-08013 Barcelona (ES); SUÑÉ NEGRE, José Mª, E-08013 Barcelona (ES)
(74) Representative: Gislon, Gabriele
(86) International application number: PCT/ES2006/000581
(87) International publication number: WO 2007/051877

(56) References cited:
- EP-A- 1 552 851
- WO-A-02/45693
- WO-A1-93/10782
- WO-A1-99/21534
- WO-A2-2005/079752
- DATABASE WPI Week 200724 Thomson Scientific, London, GB; AN 2006-555213 XP002511215 & KR 100 574 088 B1 (MEDITECH KOREA PHARM CO LTD) 27 April 2006 (2006-04-27)
- A. R. Gennaro: "Remington: The Science and Practice of Pharmacy", 2000 page 656, 884,
- "Handbook of pharmaceutical excipients", 2003 pages 222-223,
- Bast Corporation: "Technical Bulletin, Pluronic Block Copolymer NF Grades (Poloxamer NF Grades)", 2002
- "British Pharmacopoeia", 2010 pages 765-766,

## Description

### Field of the Art

The present invention relates to solid oral pharmaceutical forms of ebastine comprising a matrix consisting of a solid dispersion of said active ingredient in nonionic surfactants, such that said forms have good solubility and bioavailability properties and improved stability.

### Prior State of the Art

Ebastine is an antihistaminic and antiallergic compound corresponding to the following formula 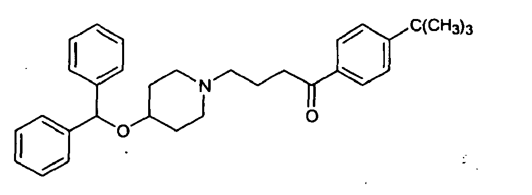 and was described in European patent application EP-A-0134124.

For the preparation of pharmaceutical forms for oral administration, said compound has the drawback of its predominantly hydrophobic character causing a low solubility thereof in water and, consequently, reducing the bioavailability of the drug.

European patent application EP-A-0575481 proposes aqueous liquid compositions of ebastine and other similar compounds for oral administration, which are free from surfactants and contain polyethylene glycol as a solubilizing agent.

Spanish patent application ES-A-2107375 describes oral liquid compositions of ebastine containing a mixture of hydroxylated carboxylic acids, nonionic surfactants and medium-chain polyols.

European patent application EP-A-0614362 describes solid oral forms of ebastine, mainly tablets, characterized in that the ebastine is micronized, such that the particles of the active ingredient have the following size characteristics:
- maximum size smaller than 200 µm,
- average granulometry between 0.5 and 15 µm,
- preferably 90% of the particles have a granulometry smaller than 25 µm,
and this makes the solid oral forms have an initial rate of dissolution greater than that obtained if ebastine is not micronized.

Micronization is an additional industrial process requiring the use of complex and high-cost machinery, which makes the active ingredient expensive. Furthermore, having a very high specific surface area in contact with the exterior, the micronized active ingredient is more sensitive to the degradation processes caused by the contact with water, air and pharmaceutical excipients.

It is therefore still necessary to have alternative solid oral forms of ebastine which do not have the mentioned drawbacks.

Patent application PCT WO02/45693 describes solid matrices in which an active ingredient is dispersed in a mixture of hydrophobic components, and said matrices are useful for preparing solid oral pharmaceutical forms, particularly tablets. Said document puts forth a very long list of several thousands of active ingredients to which said technique could be applied, and among which ebastine is mentioned as another one without any significance.

Actually, the skilled person who reads the mentioned document easily understands that the problem intended to be solved is improving the stability of certain known active ingredients as proton pump inhibitors (PPI).

The components of the matrices described in WO02/45693, except the active ingredients, are selected from:
- fatty alcohol, for example cetyl alcohol and myristyl alcohol
- triglycerides, for example glycerin tristearate,
- partial glycerides, for example glycerin monostearate,
- fatty acid esters, for example cetyl palmitate, and
- furthermore, optionally but preferably, a solid paraffin.

All of them are clearly hydrophobic components, and except in the case of partial glycerides, none of them can be considered to be a surfactant.

Partial glycerides show nonionic surfactant characteristics, but their HLBs (hydrophilic-lipophilic balance) are very low, clearly less than 10, which makes their emulsifying power not suitable for O/W (oil/water) systems, although it is suitable for W/O (water/oil) systems. For example, glyceryl monostearate shows a HLB of 2.5.

The matrices described in WO02/45693 are therefore not suitable for obtaining ebastine tablets with a fast rate of dissolution of said hydrophobic active ingredient in an aqueous medium (O/W system), and it is clear for the skilled person that the mere mention of ebastine in said document, among a list of several thousands of active ingredients, has no significance.

### Object of the Invention

The object of the present invention is a composition comprising a matrix containing a solid ebastine dispersion in nonionic surfactants, which allows obtaining oral forms of ebastine having a good performance as regards solubility and bioavailability, and at the same time showing excellent stability.

The solid oral pharmaceutical forms of ebastine which can be obtained from the mentioned matrix also form part of the object of the invention.

The ebastine tablets comprising said matrix particularly form part of the object of the invention.

### Description of the Invention

The authors of the present invention have discovered that solid oral pharmaceutical forms of ebastine can be prepared, without needing of using micronized ebastine, from a composition comprising a matrix containing ebastine dispersed in solid phase in a certain type of nonionic surfactants.

The matrices object of the invention comprise:
- (i): from 10% to 90% by weight of ebastine, and
- (ii): from 10% to 90% by weight of one or more pharmaceutically acceptable nonionic surfactants having a HLB (hydrophilic-lipophilic balance) comprised between 10 and 20 and a melting point comprised between 30° C and 70° C,
wherein ebastine is dispersed, in a solid phase, in the nonionic surfactant.

Ebastine is not limited by the particle size and is preferably in a proportion by weight comprised between 20% and 75%, more preferably between 30% and 70%.

The pharmaceutically acceptable nonionic surfactants mainly consist of polyols partially esterified with fatty acids, alkoxylated or non-alkoxylated, and mixtures thereof or mixtures thereof with glycerides or other components, and their description is within the reach of the skilled person in the usual handbooks and reference books on pharmaceutical technology, for example in the Handbook of Pharmaceutical Excipients, Fourth Edition 2003, Ed. Pharmaceutical Press, as well as in very widespread commercial catalogs.

As has already been indicated, for the purposes of the present invention, it is important that the selected nonionic surfactants have a HLB comprised between 10 and 20 and a melting point (drop point or softening point) comprised between 30° C and 70° C.

The following commercially accessible products are preferred as nonionic surfactants: GELUCIRE 50/13 and 44/14, POLYSORBATE 61 and 65 (TWEEN 61 and 65), BRIJ 58 and 76, MYRJ 59, HODAG 154-S (PEG 32 distearate) and 602-S (PEG 150 distearate), or mixtures thereof. GELUCIRE 50/13 is especially preferred.

The matrix preferably contains from 25% to 80% by weight of nonionic surfactants, more preferably from 30% to 70% by weight.

The matrix is obtained by melting, under stirring, the nonionic surfactant or mixture of nonionic surfactants and ebastine at a temperature comprised between 65° C and 90° C, preferably between 80° C and 90° C. Once homogenized, the molten mixture is allowed to cool until a temperature of preferably less than 0° C, and the cooled solid mass is subjected to grinding and subsequent sieving in conventional equipment.

It is preferred to first heat the nonionic surfactant or mixture of nonionic surfactants until a temperature at which the surfactant is molten and then add ebastine with moderate stirring and continue heating until reaching a temperature comprised between 75° C and 85° C.

It is convenient to cool the mixture until a temperature that is low enough to allow a correct and effective grinding. For example, the mixture can be cooled to a temperature comprised between 0° C and -20° C before grinding.

If desired, the mass can be first cooled until room temperature (about 20° C) and a first trituration can be carried out until reaching particles of about 1 cm, which are later cooled to between -10° C and -20° C for 10-12 hours and subsequently subjected to grinding and sieving until reaching the desired particle size.

A matrix in the form of a powdery or granular solid is thus obtained, from which oral pharmaceutical forms can be obtained by means of mixing with other pharmaceutical excipients and applying conventional Galenic techniques, which oral pharmaceutical forms can mainly consist of granulates, capsules and tablets, for example.

Said forms have a profile for releasing the active ingredient in an aqueous medium at pH 2.0 that is very similar to that obtained with commercial tablets containing micronized ebastine, even somewhat improved, showing in addition improved stability as regards the degradation of the active ingredient when they are subjected to stability tests under different temperature and relative humidity conditions.

The selection of the additional excipients is not critical and will depend on the selected pharmaceutical form and on the Galenic technique to be used. All of this only represents routine work for the skilled person, taking into account his common general knowledge and the usual handbooks and reference books in pharmaceutical technology such as, among others, the aforementioned Handbook of Pharmaceutical excipients, Remington (The Science and Practice of Pharmacy), 20th Ed., etc.

Tablets are preferred among oral pharmaceutical forms, which tablets can be obtained by mixing and homogenizing matrices object of the invention with suitable excipients, optionally if desired, the components can then be partially or completely granulated, and finally, the compression is carried out in suitable conventional machinery.

In the event that steps of completely or partially granulating the components are included, it is preferable for said granulation to be carried out in dry conditions.

The compression can be carried out on completely or partially granulated mixtures of components, or on the non-granulated components, i.e. by means of the direct compression technique that is well known by the skilled person. Tablets obtained by means of direct compression are preferred.

Once the tablet has been obtained, if desired, an outer layer of protective coating can be applied using also conventional techniques, for example by means of coating or spraying.

Diluents, disintegrants, lubricants, antiadherents, sweeteners, flavor enhancers, flavoring agents, opacifiers, etc. can be mentioned among the suitable excipients for preparing the tablets containing the matrices of the invention.

Diluents are excipients facilitating the compression of powdery materials and providing resistance to the tablets. Microcrystalline cellulose, lactose, dicalcium phosphate, PVP (polyvinylpyrrolidone), hydroxypropyl cellulose (HPC), pregelatinized starch dry flow starch and mixtures thereof, for example, can be used as suitable diluents. Preferred diluents for the purpose of the present invention are microcrystalline cellulose (AVICEL 102, for example), lactose monohydrate, dicalcium phosphate (anhydrous or dehydrate) and lactose/PVP mixtures (LUDIPRES, for example).

Disintegrants are excipients causing a quick breaking of the tablet when it is introduced in an aqueous medium, and also a quick disgregation of the granules, such that the active ingredient is quickly released. Different types of starch and energetic disintegrants, such as crospovidone, croscarmellose sodium, sodium starch glycolate and polymers derived from acrylic acid can be mentioned among the disintegrants.

Lubricants and antiadherents are excipients reducing tensions between particles and favoring the formation of the tablet, and they also prevent the adhesion of the particles. Talc, stearic acid alkaline earth salts, especially magnesium and calcium stearates, stearic acid, PEG 4000 and 6000 and stearyl fumarate can be mentioned among them. One of the most used antiadherents is colloidal silica.

The formulations of this invention can further contain sweeteners, flavoring agents and flavor enhancers for the purpose of achieving suitable organoleptic characteristics (flavor and taste) which are acceptable for patients. Sodium saccharin, aspartame, mannitol, xylitol, sucrose, sorbitol and ammonium glycyrrhizinate can be mentioned among the sweeteners and fruit and plant flavors, for example orange, anise, mint, etc. can be mentioned among the flavoring agents and flavor enhancers.

In the event that the tablets are provided with an outer coating, the coatings available on the market known as OPADRY, usually containing a mixture of hydroxypropylmethylcellulose (HPMC) and polyethylene glycol (MACROGOL), in addition to pigments and opacifiers such as titanium dioxide can be used. Gastrosoluble acrylic resins, such as EUDRAGIT E type resins, can be used.

When dispersible or mouth-dispersible tablets, i.e. tablets that dissolve quickly in a glass of water and allow their administration in liquid form to patients for whom it is difficult to swallow whole tablets, or tablets that dissolve in the mouth, are to be obtained, a suitable selection of the aforementioned energetic disintegrants (crospovidone, croscarmellose sodium, sodium starch glycolate and polymers derived from acrylic acid) can be carried out, increasing their proportion by weight to achieve the desired rate of disgregation.

To obtain dispersible tablets, an alternative consists of preparing effervescent tablets, in which part of the components form an alkaline granulate containing a chemical compound which can generate a gas, preferably sodium bicarbonate, whereas another part consists of an acid granulate, with citric acid for example, such that when it is put in contact with water, the gas (carbon dioxide) causing effervescence and quickly disgregating the tablet is generated.

The tablets object of the invention comprise:
(a) An amount of the matrix object of the invention, containing ebastine dispersed in solid phase in nonionic surfactants, which is enough to provide an effective unit dose of ebastine,
(b) At least one pharmaceutically acceptable excipient.

The tablets preferably comprise at least one diluent excipient selected from microcrystalline cellulose, lactose monohydrate, dicalcium phosphate (anhydrous or dehydrate) and lactose/PVP mixtures, or mixtures thereof.

The tablets also preferably comprise at least one disintegrant selected from crospovidone, croscarmellose sodium, sodium starch glycolate and polymers derived from acrylic acid.

The tablets also preferably comprise magnesium stearate as a lubricant.

Tablets unitarily comprising the following are especially preferred:
(a) from 30 to 50 mg of a solid matrix containing between 30% and 70% by weight of ebastine dispersed in a nonionic surfactant or mixture of nonionic surfactants, having a HLB comprised between 10 and 20 and a melting point comprised between 30° C and 70° C,
(b) from 150 to 300 mg of microcrystalline cellulose,
(c) from 2 to 7 mg of sodium starch glycolate, and
(d) from 0.5 to 1.5 mg of magnesium stearate.

The following examples are set forth below for the purposes of sufficiently completing the previous description:

### Examples

### Example 1 Obtaining a matrix of ebastine dispersed in GELUCIRE 50/13

15.0 g of GELUCIRE 50/13 were heated at 70° C until it had melted completely and then 20.0 g of ebastine were incorporated, with moderate stirring, and it was heated until 85° C. Once all the mass had melted and had a homogeneous appearance, it was poured in a siliconized tray, such that the mass formed a layer with a thickness of 0.5 cm, and the tray was then introduced in the refrigerator at -15° C for 10 to 12 hours.

The cooled mass was ground in a mill with a 6 mm opening mesh, and was stored until the time of its use for preparing the pharmaceutical form. A matrix containing 57% by weight of ebastine and 43% by weight of GELUCIRE 50/13 was thus obtained.

GELUCIRE 50/13 is a commercial nonionic surfactant consisting of a mixture of glycerides and fatty acid esters with polyethylene glycol, having a HLB of 13 and a melting point comprised between 47° C and 51° C (V-shaped capillary tube).

### Examples 2 to 16 Obtaining matrices of ebastine dispersed in different nonionic surfactants

The matrices of ebastine shown in table 1 below were obtained in a manner similar to that descried in Example 1.

**Table 1**

| Example (Matrix) | Ebastine (% by weight) | Nonionic surfactant (% by weight and identification) |
|---|---|---|
| 2 | 57% | 43% of PEG 150 distearate |
| 3 | 57% | 43% of TWEEN 65 |
| 4 | 57% | 43% of MYRJ 45 |
| 5 | 57% | 43% of BRIJ 58 |
| 6 | 57% | 43% of BRIJ 76 |
| 7 | 25% | 75% of GELUCIRE 44/14 |
| 8 | 50% | 50% of GELUCIRE 44/14 |
| 9 | 33% | 67% of GELUCIRE 44/14 |
| 10 | 40% | 60% of GELUCIRE 44/14 |
| 11 | 63% | 37% of GELUCIRE 44/14 |
| 12 | 33% | 67% of GELUCIRE 50/13 |
| 13 | 40% | 60% of GELUCIRE 50/13 |
| 14 | 50% | 50% of GELUCIRE 50/13 |
| 15 | 67% | 33% of GELUCIRE 50/13 |
| 16 | 61.5% | 38.5% of GELUCIRE 50/13 |

PEG 150 distearate is a nonionic surfactant which can acquired on the market, for example with the reference HODAG 602-S, having a HLB of 18.4 and a melting point comprised between 53° C and 57° C.

TWEEN 65 is a commercial nonionic surfactant formed by fatty acid esters with polyoxyethylenated sorbitan. It has a HLB of 10.5 and a melting point comprised between 53° C and 57° C.

MYRJ 45 is a commercial nonionic surfactant consisting of polyethylene glycol 100 monostearate. It has a HLB of 18.8 and a melting point of about 46° C.

BRIJ 58 and 76 are commercial nonionic surfactants. BRIJ 58 is a polyoxyl 20 cetyl ether with a HLB of 15.7 and a melting point of 38° C. BRIJ 76 is a polyoxyl 10 cetyl ether with a HLB of 12.4 and a melting point of 38° C.

GELUCIRE 44/14 is a commercial nonionic surfactant similar to GELUCIRE 50/13, having a HLB of 14 and a melting point comprised between 42° C and 46° C.

### Example 17 Ebastine tablets obtained with the matrix of Example 1

Tablets having the following composition per tablet were obtained from the matrix of Example 1:

| | |
|---|---|
| Matrix (57% ebastine and 43% GELUCIRE 50/13) | 35.0 mg |
| AVICEL 102 (microcrystalline cellulose) | 219.5 mg |
| Sodium starch glycolate | 4.5 mg |
| Magnesium stearate | 1.0 mg |

The tablets were obtained as follows:

The matrix obtained in Example 1 and AVICEL 102 were mixed and sieved by means of an oscillating granulator-sieve provided with a 0.8 mm mesh. The mixture was stirred for 5 minutes and then sodium starch glycolate was added over 10 minutes. Magnesium stearate was then added and it was mixed for 1 more minute.

The mixture thus obtained was compressed in a rotary compression machine provided with biconvex and smooth punches with a diameter of 9 mm.

Two batches of tablets, A and B, with the same composition and manufactured independently according to the described method, were subjected to an *in vitro* dissolution profile test in aqueous medium at pH 2.0. A batch of tablets of the commercial product EBASTEL FORTE, containing micronized ebastine, was also subjected to said test in the same conditions.

The ebastine content in solution was analyzed over time and the result shown in Table 2 below were obtained:

**Table 2**

| Time (minutes) | Batch A (ebastine in solution in %) | Batch B (ebastine in solution in %) | EBASTEL FORTE (ebastine in solution in %) |
|---|---|---|---|
| 5 | 88.6 | 76.4 | 78.9 |
| 10 | 96.3 | 94.4 | 88.0 |
| 15 | 98.4 | 97.4 | 91.2 |
| 20 | 99.3 | 99.1 | 93.0 |
| 30 | 100.0 | 99.5 | 94.1 |
| 45 | 100.8 | 100.5 | 95.2 |
| 60 | 101.2 | 100.8 | 95.8 |

As observed, taking into account the typical errors of the method, it can be affirmed that the tablets according to the invention show dissolution profiles that are similar to that of the tablets of the prior state of the art containing micronized ebastine, being even somewhat better as regards the quickness of dissolution.

A batch of the tablets obtained according to that described in this example and a batch of tablets of the commercial product EBASTEL FORTE were subjected to stability tests in different temperature and relative humidity conditions. The degradation level over time was determined by means of the total impurity content, referred to ebastine, analyzed by means of HPLC techniques.

The results are set forth in Table 3 below:

**Table 3**

| Conditions | Time | Example 17 (% of total impurities with respect to ebastine) | EBASTEL FORTE (% of total impurities with respect to ebastine) |
|---|---|---|---|
| 25° C 60% RH | Start | 0.07 | 0.22 |
| | 3 months | 0.12 | 0.47 |
| | 6 months | 0.07 | 0.64 |
| 30° C 65% RH | Start | 0.07 | 0.22 |
| | 3 months | 0.06 | 0.56 |
| | 6 months | 0.04 | 0.77 |
| 40° C 75% RH | Start | 0.07 | 0.22 |
| | 3 months | 0.06 | 0.57 |
| | 6 months | 0.06 | 0.60 |

As observed in Table 3, in the different tested conditions, the ebastine contained in the tablets of the invention remains stable over time, without undergoing degradations, and the total impurity content does not increase. On the contrary, in the tablets of the state of the art, ebastine undergoes a progressive increase in the total impurity content, which indicates greater degradation and less stability.

### Examples 18 to 32 Other tablets according to the invention

By following methods similar to that described in Example 17, ebastine tablets the composition of which is shown below are prepared from the matrices of ebastine of Examples 2 to 16:

### Example 18

| | |
|---|---|
| Matrix of Example 2 | 35.0 mg |
| AVICEL 102 (microcrystalline cellulose) | 219.5 mg |
| Sodium starch glycolate | 4.5 mg |
| Magnesium stearate | 1.0 mg |

### Example 19

| | |
|---|---|
| Matrix of Example 3 | 35.0 mg |
| AVICEL 102 (microcrystalline cellulose) | 219.5 mg |
| Sodium starch glycolate | 4.5 mg |
| Magnesium stearate | 1.0 mg |

### Example 20

| | |
|---|---|
| Matrix of Example 4 | 35.0 mg |
| AVICEL 102 (microcrystalline cellulose) | 219.5 mg |
| Sodium starch glycolate | 4.5 mg |
| Magnesium stearate | 1.0 mg |

### Example 21

| | |
|---|---|
| Matrix of Example 5 | 35.0 mg |
| AVICEL 102 (microcrystalline cellulose) | 219.5 mg |
| Sodium starch glycolate | 4.5 mg |
| Magnesium stearate | 1.0 mg |

### Example 22

| | |
|---|---|
| Matrix of Example 6 | 35.0 mg |
| AVICEL 102 (microcrystalline cellulose) | 219.5 mg |
| Sodium starch glycolate | 4.5 mg |
| Magnesium stearate | 1.0 mg |

### Example 23

| | |
|---|---|
| Matrix of Example 7 | 40.0 mg |
| Lactose monohydrate (GRANULAC 200) | 64.0 mg |
| PVP (PVPK 25) | 20.0 mg |
| Sodium starch glycolate | 6.0 mg |

### Example 24

| | |
|---|---|
| Matrix of Example 8 | 20.0 mg |
| Lactose monohydrate (GRANULAC 200) | 99.5 mg |
| Sodium starch glycolate | 10.0 mg |
| Magnesium stearate | 0.5 mg |

### Example 25

| | |
|---|---|
| Matrix of Example 9 | 30.0 mg |
| Dicalcium phosphate dihydrate | 82.5 mg |
| Crospovidone | 6.5 mg |
| Sodium starch glycolate | 10.5 mg |
| Magnesium stearate | 0.5 mg |

### Example 26

| | |
|---|---|
| Matrix of Example 10 | 25.0 mg |
| Lactose monohydrate (Granulac 200) | 30.0 mg |
| Lactose monohydrate (Fast flo) | 194.8 mg |
| Crospovidone | 46.2 mg |
| Sodium starch glycolate | 33.0 mg |
| Magnesium stearate | 1.0 mg |

### Example 27

| | |
|---|---|
| Matrix of Example 11 | 16.0 mg |
| Lactose monohydrate (GRANULAC 200) | 10.0 mg |
| Lactose monohydrate (Fast flo) | 68.3 mg |
| PVP (PVPK 25) | 20.0 mg |
| Sodium starch glycolate | 13.0 mg |
| Magnesium stearate | 0.7 mg |

### Example 28

| | |
|---|---|
| Matrix of Example 12 | 60.0 mg |
| AVICEL 102 (microcrystalline cellulose) | 123.6 mg |
| Sodium starch glycolate | 26.0 mg |
| Crospovidone | 40.0 mg |
| Sodium lauryl sulfate | 10.4 mg |

### Example 29

| | |
|---|---|
| Matrix of Example 13 | 50.0 mg |
| AVICEL 102 (microcrystalline cellulose) | 133.6 mg |
| Sodium starch glycolate | 26.0 mg |
| Crospovidone | 40.0 mg |
| Sodium lauryl sulfate | 10.4 mg |

### Example 30

| | |
|---|---|
| Matrix of Example 14 | 40.0 mg |
| AVICEL 102 (microcrystalline cellulose) | 143.6 mg |
| Sodium starch glycolate | 26.0 mg |
| Crospovidone | 40.0 mg |
| Sodium lauryl sulfate | 10.4 mg |

### Example 31

| | |
|---|---|
| Matrix of Example 15 | 30.0 mg |
| AVICEL 102 (microcrystalline cellulose) | 162.7 mg |
| Sodium starch glycolate | 26.0 mg |
| Crospovidone | 40.0 mg |
| Magnesium stearate | 1.3 mg |

### Example 32

| | |
|---|---|
| Matrix of Example 16 | 32.5 mg |
| AVICEL 102 (microcrystalline cellulose) | 160.2 mg |
| Sodium starch glycolate | 26.0 mg |
| Crospovidone | 40.0 mg |
| Magnesium stearate | 1.3 mg |

The tablets of Examples 18 to 32 show good solubility properties and also good stability.

## Claims

1. A composition useful for preparing solid oral pharmaceutical forms of ebastine, said composition being in the form of a matrix comprising:
(i) from 10% to 90% by weight of ebastine, and
(ii) from 10% to 90% by weight of one or more pharmaceutically acceptable nonionic surfactants having a HLB (hydrophilic-lipophilic balance) comprised between 10 and 20 and a melting point comprised between 30° C and 70° C,
wherein ebastine is dispersed, in a solid phase, in the nonionic surfactant.

2. A composition according to claim 1, **characterized in that** it comprises between 20% and 75% by weight of ebastine and from 25% to 80% by weight of nonionic surfactant.

3. A composition according to claim 2, **characterized in that** it comprises between 30% and 70% by weight of ebastine and from 30% to 70% of nonionic surfactant.

4. A composition according to claims 1 to 3, **characterized in that** the nonionic surfactant is selected from the group consisting of the commercial products GELUCIRE 50/13 and 44/14, POLYSORBATE 61 and 65 (TWEEN 61 and 65), BRIJ 58 and 76, MYRJ 59, HODAG 154-S (PEG 32 distearate) and 602-S (PEG 150 distearate), and mixtures thereof.

5. A composition according to claim 4, **characterized in that** the nonionic surfactant is GELUCIRE 50/13.

6. A solid pharmaceutical form of ebastine for oral administration comprising the compositions of claims 1 to 5 and at least one pharmaceutically acceptable excipient.

7. An ebastine tablet comprising:
(a) an amount of the compositions of claims 1 to 5 which is enough to provide an effective unit dose of ebastine
(b) at least one pharmaceutically acceptable excipient.

8. A tablet according to claim 7, **characterized in that** it comprises at least one diluent excipient selected from microcrystalline cellulose, lactose monohydrate, dicalcium phosphate (anhydrous or dihydrated) and lactose/PVP mixtures, or mixtures thereof.

9. A tablet according to claims 7 and 8, **characterized in that** it comprises at least one disintegrant selected from crospovidone, croscarmellose sodium, sodium starch glycolate and polymers derived from acrylic acid.

10. A tablet according to claims 7 to 9, **characterized in that** it comprises magnesium stearate as a lubricant.

11. A tablet comprising:
(a) from 30 to 50 mg of a solid matrix containing between 30% and 70% by weight of ebastine dispersed in a nonionic surfactant or mixture of nonionic surfactants, having a HLB comprised between 10 and 20 and a melting point comprised between 30° C and 70° C,
(b) from 150 to 300 mg of microcrystalline cellulose,
(c) from 2 to 7 mg of sodium starch glycolate, and
(d) from 0.5 to 1.5 mg of magnesium stearate.

12. A tablet according to claims 7 to 10 and 11, **characterized in that** it has an outer layer of protective coating.

13. A tablet according to any of claims 7 to 12, **characterized in that** it is a dispersible or mouth-dispersible type tablet.

14. The use of the compositions of claims 1 to 5 for preparing solid pharmaceutical forms of ebastine for oral administration.

15. The use according to claim 14, **characterized in that** the pharmaceutical form is a tablet.

## Patentansprüche

1. Zusammensetzung geeignet für die Herstellung fester oraler pharmazeutischer Formen von Ebastine, wobei die genannte Zusammensetzung in Form einer Matrix ist, welche:
(i) von 10 bis 90 Gew.-% Ebastine, und
(ii) von 10 bis 90 Gew.-% eines oder mehrerer pharmazeutisch akzeptabler nichtionischer Tenside, welche ein HLB (Hydrophil-Lipophil- Gleichgewicht) aufweisen, das zwischen 10 und 20 liegt, und einen Schmelzpunkt, der zwischen 30°C und 70°C liegt,
beinhaltet, wobei Ebastine, in einer festen Phase, im nichtionischen Tensid dispergiert ist.

2. Zusammensetzung nach Anspruch 1, **dadurch gekennzeichnet, dass** sie zwischen 20 und 75 Gew.-% Ebastine und von 25 bis 80 Gew.-% des nichtionischen Tensids beinhaltet.

3. Zusammensetzung nach Anspruch 2, **dadurch gekennzeichnet, dass** sie zwischen 30 und 70 Gew.-% Ebastine und von 30 bis 70 Gew.-% des nichtionischen Tensids beinhaltet.

4. Zusammensetzung nach den Ansprüchen 1 bis 3, **dadurch gekennzeichnet, dass** das nichtionische Tensid von der Gruppe gewählt wird, die aus den Handelsprodukten GELUCIRE 50/13 und 44/14, POLYSORBATE 61 und 65 (TWEEN 61 und 65), BRIJ 58 und 76, MYRJ 59, HODAG 154-S (PEG-32 Distearat) und 602-S (PEG-150 Distearat), und Mischungen derselben, besteht.

5. Zusammensetzung nach Anspruch 4, **dadurch gekennzeichnet, dass** das nichtionische Tensid GELUCIRE 50/13 ist.

6. Feste pharmazeutische Form von Ebastine für orale Verabreichung, welche die Zusammensetzungen nach den Ansprüchen 1 bis 5 und zumindest einen pharmazeutisch akzeptablen Hilfsstoff beinhaltet.

7. Ebastine Tablette, welche:
(a) eine Menge der Zusammensetzungen nach den Ansprüchen 1 bis 5, die genug ist, um eine wirksame Einzeldosis von Ebastine bereitzustellen
(b) zumindest einen pharmazeutisch akzeptablen Hilfsstoff,
beinhaltet.

8. Tablette nach Anspruch 7, **dadurch gekennzeichnet, dass** sie zumindest einen verdünnenden Hilfsstoff gewählt aus mikrokristaline Zellulose, Laktose-Monohydrat, Dicalciumphosphat (wasserfrei oder hydriert) und Mischungen aus Laktose/PVP, oder Mischungen derselben, beinhaltet.

9. Tablette nach den Ansprüchen 7 und 8, **dadurch gekennzeichnet, dass** sie zumindest ein Sprengmittel gewählt aus Crospovidon, Natrium Croscarmellose, Natrium-Stärke-Glykolat und aus Acrylsäure abgeleitete Polymere, beinhaltet.

10. Tablette nach den Ansprüchen 7 bis 9, **dadurch gekennzeichnet, dass** sie Magnesiumstearat als Gleitmittel beinhaltet.

11. Tablette, welche:
(a) von 30 bis 50 mg einer festen Matrix, die zwischen 30 und 70 Gew.-% Ebastine beinhaltet, die in einem nichtionischen Tensid oder einer Mischung aus nichtionischen Tensiden dispergiert ist, welche ein HLB das zwischen 10 und 20 liegt und einen Schmelzpunkt, der zwischen 30°C und 70°C liegt, aufweisen,
(b) von 150 bis 300 mg mikrokristaline Zellulose,
(c) von 2 bis 7 mg Natrium-Stärke-Glykolat, und
(d) von 0,5 bis 1,5 mg Magnesiumstearat,
beinhaltet.

12. Tablette nach den Ansprüchen 7 bis 10 und 11, **dadurch gekennzeichnet, dass** sie eine Außenschicht einer schützenden Beschichtung aufweist.

13. Tablette nach einem der Ansprüche 7 bis 12, **dadurch gekennzeichnet, dass** sie eine dispergierbare Tablette ist oder eine Tablette vom Typ, die im Mund dispergierbar ist.

14. Verwendung der Zusammensetzungen nach den Ansprüchen 1 bis 5, zur Herstellung fester pharmazeutischer Formen von Ebastine für orale Verabreichung.

15. Verwendung nach Anspruch 14, **dadurch gekennzeichnet, dass** die pharmazeutische Form eine Tablette ist.

## Revendications

1. Une composition utile pour préparer des formes pharmaceutiques orales solides d'ébastine, cette composition étant sous forme d'une matrice comportant:
(i) de 10% à 90% du poids d'ébastine, et
(ii) de 10% à 90% du poids d'un ou plusieurs tensioactifs non ioniques
acceptables du point de vue pharmaceutique ayant un HLB (équilibre hydrophile-lipophile) compris entre 10 et 20 et un point de fusion compris entre 30° C et 70° C où l'ebastine est dispersée, en phase solide, dans l'agent tensio-actif non ionique.

2. Une composition conformément à la revendication 1, **caractérisée en ce qu'**elle comporte entre 20% et 75% du poids d'ébastine et 25% à 80% du poids d'agent tensio-actif non ionique.

3. Une composition conformément à la revendication 2, **caractérisée en ce qu'**elle comporte 30% à 70% du poids d'ébastine et 30% à 70% d'agent tensio-actif non ionique.

4. Une composition conformément aux revendications 1 à 3, **caractérisée en ce que** l'agent tensio-actif non ionique est sélectionné d'une groupe consistant en les produits commerciaux GELUCIRE 50/13 et 44/14, POLYSORBATE 61 et 65 (TWEEN 61 et 65), BRIJ 58 et 76, MYRJ 59, HODAG 154-S (PEG 32 distéarate) et 602-S (PEG 150 distéarate) et leurs mélanges.

5. Une composition conformément à la revendication 4, **caractérisée en ce que** l'agent tensio-actif non ionique est le GELUCIRE 50/13.

6. Une forme pharmaceutique solide d'ébastine pour l'administration orale comportant les compositions des revendications 1 à 5 et au moins un excipient acceptable du point de vue pharmaceutique.

7. Une tablette d'ébastine comportant:
(a) une quantité des compositions des revendications 1 à 5 suffisante pour offrir une dose unitaire efficace d'ébastine.
(b) au moins un excipient acceptable du point de vue pharmaceutique.

8. Une tablette conformément à la revendication 7, **caractérisé en ce qu'**elle comporte au moins un excipient diluant sélectionné entre la cellulose microcristalline, le monohydrate de lactose, le phosphate dicalcique (anhydre ou dihydraté) et lactose/mélanges de PVP ou leurs mélanges.

9. Une tablette conformément aux revendications 7 et 8, **caractérisée en ce qu'**elle comporte au moins un agent désagrégeant sélectionné entre le crospovidone, sodium de croscarmellose, glycolate d'amidon de sodium et des polymères dérivés de l'acide acrylique.

10. Une tablette conformément aux revendications 7 et 9 **caractérisée en ce qu'**elle comporte du stéréate de magnésium comme lubrifiant.

11. Une tablette comportant:
(a) de 30 à 50 mg de matrice solide contenant de 30% à 70% du poids d'ébastine dispersée dans un agent tensio-actif non ionique ou un mélange d'agents tensio-actifs non ioniques ayant un HLB compris entre 10 et 20 et un point de fusion compris entre 30° et 70°C,
(b) de 150 à 300 mg de cellulose microcristalline.
(c) de 2 à 7 mg de glycolate d'amidon de sodium, et
(d) de 0,5 à 1,5 mg de stéréate de magnésium

12. Une tablette conformément aux revendications 7 à 10 et 11, **caractérisée en ce qu'**elle possède une couche extérieure de revêtement de protection.

13. Une tablette conformément à une quelconque des revendications 7 à 12, **caractérisée en ce qu'**il s'agit d'une tablette du genre pouvant être dispersée ou pouvant être dispersée dans la bouche.

14. L'usage des compositions de revendications 1 à 5 pour préparer des formes pharmaceutiques solides d'ébastine pour l'administration orale.

15. L'usage conformément à la revendication 14, **caractérisé en ce que** la forme pharmaceutique est d'une tablette.
